(19) **Europäisches Patentamt / European Patent Office / Office européen des brevets**

(11) **EP 2 561 502 B1**

(12) **EUROPEAN PATENT SPECIFICATION**

(45) Date of publication and mention
of the grant of the patent:
**25.09.2019 Bulletin 2019/39**

(51) Int Cl.:
*G09B 19/16* (2006.01)    *B60K 31/00* (2006.01)
*B60K 31/18* (2006.01)    *B60W 40/08* (2012.01)
*B60W 50/08* (2012.01)    *A61B 5/18* (2006.01)
*B60W 30/02* (2012.01)

(21) Application number: **11772321.3**

(22) Date of filing: **18.04.2011**

(86) International application number:
**PCT/SE2011/050469**

(87) International publication number:
**WO 2011/133091 (27.10.2011 Gazette 2011/43)**

(54) **ASSESSMENT METHOD AND SYSTEM PERTAINING TO ACCELERATION**

BESCHLEUNIGUNGSMESSVERFAHREN UND -SYSTEM

PROCÉDÉ ET SYSTÈME D'ÉVALUATION APPLIQUÉE À L'ACCÉLÉRATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.04.2010 SE 1050394**

(43) Date of publication of application:
**27.02.2013 Bulletin 2013/09**

(73) Proprietor: **Scania CV AB
151 87 Södertälje (SE)**

(72) Inventors:
• **BREDBERG, Linus
SE-126 39 Hägersten (SE)**
• **ANDERSSON, Jonny
SE-151 68 Södertälje (SE)**

• **JERHAMMAR, Andreas
SE-146 37 Tullinge (SE)**

(74) Representative: **Scania CV AB
Patents, GP 117kv
151 87 Södertälje (SE)**

(56) References cited:
| EP-A1- 1 811 481 | EP-A1- 1 811 481 |
| EP-A2- 2 105 338 | WO-A1-2007/139491 |
| WO-A1-2007/139491 | WO-A1-2008/078088 |
| WO-A1-2008/078088 | GB-A- 2 443 645 |
| GB-A- 2 443 645 | US-A1- 2002 091 473 |
| US-A1- 2005 021 222 | US-A1- 2007 276 582 |
| US-A1- 2007 276 582 | US-A1- 2008 255 722 |
| US-A1- 2010 055 649 | US-A1- 2010 055 649 |

## Description

### Field of the invention

The present invention relates to a method and a system for assessing a driver's acceleration behaviour according to the preambles of the independent claims.

### Background to the invention

Defensive driving is often synonymous with fuel-efficient driving. It also contributes to maintaining good traffic flow, greater comfort for any passengers carried and safe carriage of any load carried. What directly contributes to both comfort and safety is how the person driving the vehicle accelerates. The acceleration may be both longitudinal, i.e. in the vehicle's direction of movement, and lateral, i.e. in the vehicle's sideways direction. The vehicle's acceleration may of course also be a combination of longitudinal and lateral acceleration.

High lateral accelerations may not only cause passengers discomfort and risk of falling but also present a safety risk due to uncontrolled movement of unsecured objects. In a truck context, high lateral accelerations may result in cargo shifting or tilting and thereby sustaining damage. In the worst case, high lateral acceleration may also cause a traffic accident. Low lateral acceleration also helps to reduce tyre wear.

US 2008/0015754 describes a system adapted to automatically compensating for sudden changes in a vehicle's lateral acceleration. The lateral acceleration is estimated while the vehicle is in motion, and if it exceeds a predetermined value a control signal adapted to countering the adverse sideways force is generated.

US 2009/0248240 describes a system which measures a vehicle's lateral acceleration and passes the information on to the driver. He/she may then decide whether to reduce, maintain or increase the vehicle's speed in order to be able as soon as possible to drive it in a controlled way. The system is for example used to train racing drivers. The information may also be saved for subsequent analysis.

US2009/0319129 describes a method for assisting a driver during driving. The method calculates the vehicle's maximum permissible lateral acceleration and recommends a longitudinal speed to the driver on the basis of that value.

GB2443645 describes a portable driving style monitoring device for use in a vehicle. Movement information is used to provide driver training by advising the driver when programmed thresholds corresponding to a safe driving style are approached.

The object of the invention is to improve a driver's acceleration behaviour and in particular to encourage him/her to improve his/her acceleration behaviour.

### Summary of the invention

The object described above is achieved by an assessment method for assessing acceleration behaviour of a driver of a vehicle. The method comprises determining the vehicle's acceleration a, calculating one or more threshold values for the vehicle's acceleration which depend on at least one situation-specific parameter, comparing the vehicle's acceleration a with said one or more threshold values, and generating on the basis of the comparison a grading signal related to said calculated one or more threshold values.

According to another aspect, the object is achieved by an assessment system for assessing acceleration behaviour of a driver of a vehicle. The system comprises at least one sensor unit adapted to delivering an acceleration signal which indicates the vehicle's acceleration a, and a calculation unit adapted to receiving said acceleration signal, calculating one or more threshold values for the vehicle's acceleration which depend on at least one situation-specific parameter, comparing the vehicle's acceleration a with said one or more threshold values, and generating on the basis of the comparison a grading signal related to said calculated one or more threshold values.

The system further comprises an output unit adapted to presenting information from the grading signal to the driver. The grading signal may according to an embodiment be sent to an external unit for presentation of information to, for example, the vehicle owner.

The method and the system provide the driver in a constructive way with encouragement to adapt the vehicle's speed in due time to bends or roundabouts ahead. Comfort for any passengers carried may then be improved while at the same time the risk of damage to any cargo may be reduced. The method and the system also help to reduce wear on tyres and other vehicle components, which may result in less time being needed for servicing and hence in more time during which the vehicle can be used. Reducing wear also reduces environmental impact in that vehicle parts need replacing less frequently.

The driver's acceleration behaviour is assessed by being graded on a scale which depends on the situation in which the vehicle is. The vehicle's situation is therefore observed and the driver's acceleration behaviour in response to it is assessed.

The vehicle's acceleration may be divided into lateral acceleration and longitudinal acceleration. In the case of lateral acceleration, the problem comprises according to an embodiment two cases, one of them being a temporary "spike" in lateral acceleration due to a rapid steering wheel movement on a bend, also called "jerk", and the other being continuously high lateral acceleration due to taking a bend at too high a speed. In the case of longitudinal acceleration, the problem according to an embodiment is too high acceleration related to a certain speed.

Assessment of the driver's acceleration behaviour may be part of a set of categories for assessing his/her driving behaviour.

Preferred embodiments are described in the dependent claims and the detailed description.

Brief description of the attached drawings

The invention is described below with reference to the attached drawings, in which:

Figure 1 depicts an assessment system according to an embodiment of the invention.
Figure 2 is a flowchart for the assessment method according to an embodiment of the invention.
Figure 3 is a flowchart for the assessment method according to another embodiment of the invention.
Figure 4 is a diagram illustrating an example of how a rapid steering wheel movement on a bend may affect the vehicle's lateral acceleration (curve A), and what the lateral acceleration may be like in cases where the vehicle takes the bend at too high a speed (curve B).
Figure 5 illustrates in diagram form an example of the vehicle's maximum and mean lateral acceleration on a bend.
Figure 6 is a flowchart for the assessment method according to a further embodiment of the invention.
Figure 7 illustrates how the threshold values for assessment of the vehicle's longitudinal acceleration are arrived at according to an embodiment of the invention.
Figure 8 depicts examples of permissible reference acceleration values for assessing the driver's acceleration behaviour in the case of longitudinal acceleration.

Detailed description of preferred embodiments of the invention

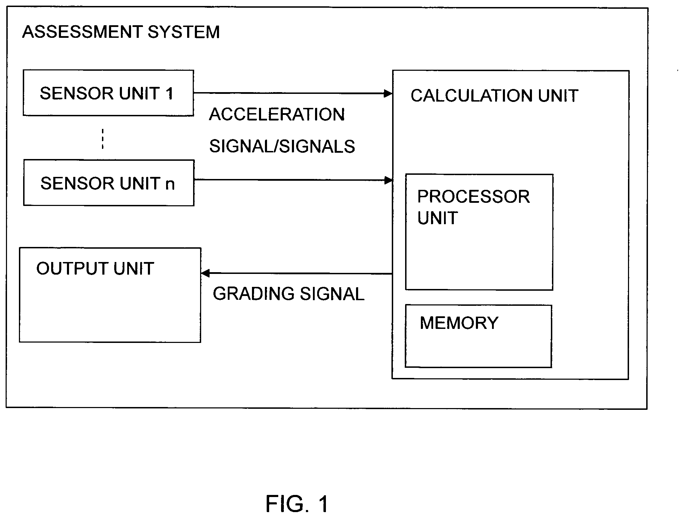

Figure 1 depicts an assessment system according to the invention which will now be explained with reference to the invention. The system comprises at least one sensor unit 1... n adapted to delivering an acceleration signal which indicates the vehicle's acceleration a. The sensor unit may for example be an accelerometer which measures the vehicle's acceleration, or a unit which measures the difference in wheel speed between the sides of the vehicle and then calculates the vehicle's lateral acceleration. The system further comprises a calculation unit adapted to receiving said acceleration signal. The calculation unit is further adapted to calculating one or more threshold values for the vehicle's acceleration which depend on at least situation-specific parameter, comparing the vehicle's acceleration with said one or more threshold values, and generating a grading signal based on the comparison. The grading signal is related to said calculated one or more threshold values.

The system comprises according to an embodiment an output unit adapted to presenting the driver with information from the grading signal. The grading signal may according to another embodiment be sent via a telematic unit in the vehicle to an external unit for presentation of the information from the grading signal to, for example, the vehicle owner. The driver may then subsequently be provided with information about his/her acceleration behaviour, e.g. via a report.

The calculation unit comprises preferably a processor unit adapted to doing calculations, comparisons etc., and one or more memories. The output unit may for example be a display in the instrument panel in the vehicle, or an audio unit which conveys hints, feedback and/or grading in the form of voice messages.

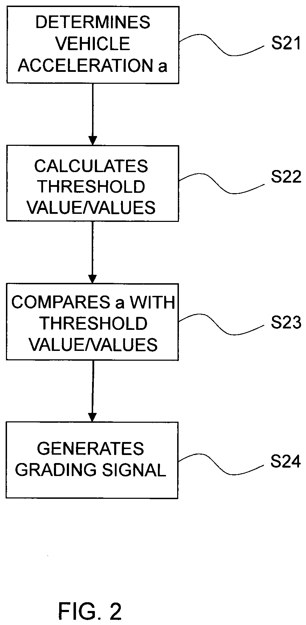

Figure 2 is a flowchart for the method according to an embodiment of the invention and comprises a first step S21 which determines the vehicle's acceleration a. A second step S22 calculates one or more threshold values for the vehicle's acceleration which depend on at least one situation-specific parameter. The threshold values preferably indicate limits for what is regarded as safe and comfortable cornering or safe and comfortable longitudinal acceleration. According to an embodiment, a situation-specific parameter is any from among the vehicle's speed, the vehicle's acceleration, the radius of curvature of the road or the arc angle of the road. The vehicle's speed is a parameter which is usually accessible via the vehicle's internal communication network, and may be received in the calculation unit in the form of a speed signal. The radius of curvature of a road may be determined by the calculation unit having access to map data concerning the vehicle's route and information about the vehicle's location, which may be obtained via a positioning unit in the vehicle, e.g. via GPS (global positioning system). This makes it possible to ascertain the radius of curvature in advance. Alternatively, the radius of curvature may be determined while the vehicle is negotiating, or after it has negotiated, the bend from, for example, the alignments of the wheels during the bend. The arc angle or edge angle of the road may for example be determined by measuring the vehicle's amount of time spent or distance travelled on a bend, i.e. the time or distance over which the vehicle's sensor unit 1. n delivers an acceleration signal which indicates lateral acceleration. If the lateral acceleration is zero, the vehicle is travelling straight ahead.

Said threshold value or values are therefore calculated on the basis of the situation in which the vehicle is. The way they change depends on one or more situation-specific parameters, e.g. the threshold values at a vehicle speed of 50 km/h may be different from those at 70 km/h.

A step S23 then compares the vehicle's acceleration a with said one or more threshold values, followed by a step S24 which generates a grading signal based on the comparison and therefore related to said calculated one or more threshold values. The grading signal therefore adapts itself to the threshold values determined for the specific situation and hence to the scopes which follow from that situation.

According to an embodiment, the information from the grading signal is presented to the driver via, for example, an output unit. The grading signal may instead or in addition be sent to an external unit for presentation of information to, for example, the vehicle owner. The information presented may for example be in the form of a numeral on a scale from 1 to 10, in which 1 is not good acceleration behaviour and 10 is very good acceleration behaviour for the situation. Another example of how the grading may be expressed is in the form of a number of symbols, e.g. stars, in which case the number of symbols which light up depends on the grading signal. If only one symbol lights up, this indicates less good acceleration behaviour, and if all of the symbols light up this indicates good acceleration behaviour. The driver thus receives continuous feedback about how well he/she accelerated in the situation and is motivated to improve his/her results. According to an embodiment, the information in the grading signals is accumulated and saved in a memory in, for example, the calculation unit to allow it to be analysed subsequently. The information may also be sent to a central unit situated externally from the vehicle for further analysis and follow-up. According to an embodiment, the method comprises, at the time when a grading signal is generated, feeding back to the driver via an output unit how he/she can improve his/her acceleration behaviour, or praising his/her acceleration behaviour. The calculation unit is then adapted to, for example, generating via the output unit a text message related to the information from the grading signal. In the event of a low grading the driver may be urged to "Reduce speed before the bend", or in the event of a high grading he/she may be congratulated by, for example, "Good cornering!". The calculation unit according to an embodiment is adapted to taking account of whether the driver brakes or accelerates on a bend, accelerates or brakes before the bend etc., in cases where there is to be feedback to the driver.

According to an embodiment, said acceleration is the vehicle's lateral acceleration $a_{lat}$. Lateral acceleration occurs mainly when the vehicle is cornering. The problem with regard to lateral acceleration comprises mainly two cases, 1 and 2. Case 1 is a temporary "spike" in lateral acceleration caused by a rapid steering wheel movement. This may for example be due to the driver not having realised that a bend was coming and consequently having had to turn the steering wheel quickly to avoid running off the road or into an incorrect lane. Case 2 is continuously high lateral acceleration due to taking a bend at too high a speed. Assessment of cornering involves according to an embodiment the fulfilment of at least one situation criterion. If at least one situation criterion is fulfilled, the calculation unit is then adapted to allowing the vehicle's lateral acceleration to be assessed. According to an embodiment, said situation criterion is any from among a lowest vehicle speed, a lowest acceleration, a largest radius of curvature or a smallest arc angle. It is thus possible to ensure that very slight bends are not assessed and also that the driver is driving in normal running conditions. Lowest vehicle speed entails the vehicle travelling at above a certain speed, e.g. 20 km/h, before and after the bend, for the driver's acceleration behaviour on the bend to have to be assessed. Largest radius of curvature entails according to an embodiment the radius being below a value which depends on the vehicle's speed before the bend. Suitable values appear for example in the Swedish National Road Administration's highway construction recommendations (VV publication 2004:80), which state limits so that the radius of curvature is below what is classified as "less good" to "good". Smallest arc angle entails according to an embodiment the arc angle exceeding a value which depends on the vehicle's speed. For example, high values are set for the arc angle at low speeds (about 70 degrees) in order to assess only bends and no slight curves. At high speeds this value may be very low or even zero. This limitation is therefore used mostly as a filter in order to disregard slight curves in urban surroundings.

We now go on to explain how case 1 and case 2 described above may be detected and assessed.

**Case 1**

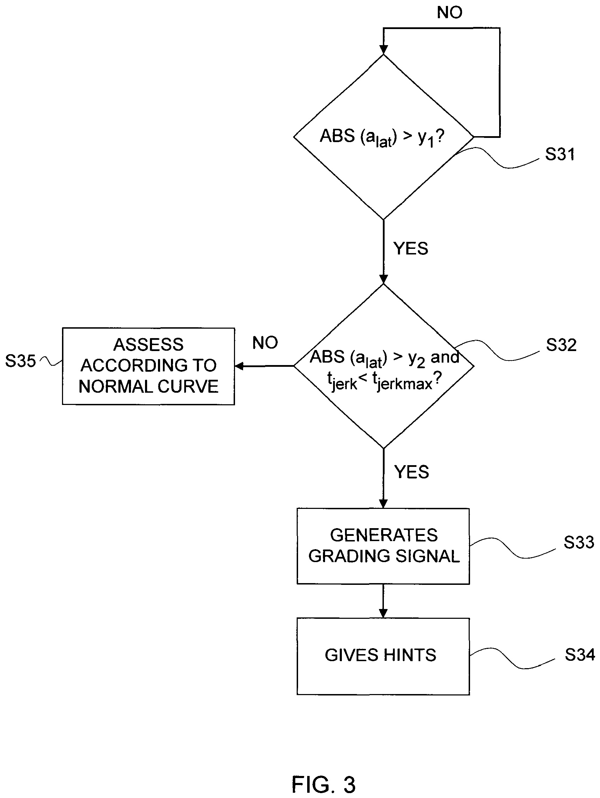

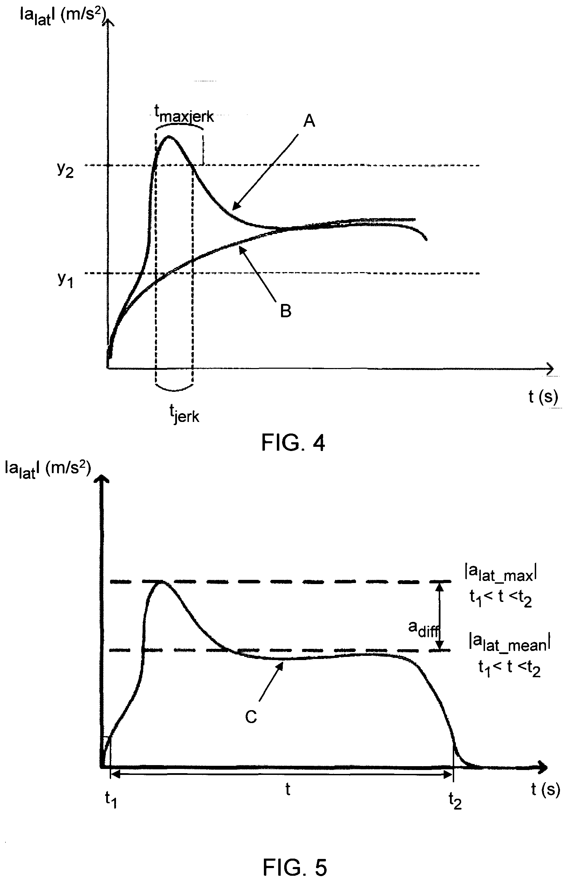

According to an embodiment, the calculation unit is adapted to determining the absolute amount for $a_{lat}$ during a predetermined period of time $t_{maxjerk}$ and comparing this value with a threshold value $y_2$, and generating a grading signal based on the comparison. Figure 3 illustrates a method for deciding whether a driver makes a rapid steering wheel movement, and curve A in the diagram in Figure 4 illustrates how a rapid steering wheel movement on a bend may affect the vehicle's lateral acceleration. Curve B illustrates the possible pattern of lateral acceleration when the speed at which the vehicle takes the bend is too high. According to Figure 3, the absolute amount for $a_{lat}$ is compared with the acceleration threshold value yi at step S31, and if $|a_{lat}|$ is greater than yi the driver's acceleration behaviour has to be assessed. The situation criterion here is therefore that a lowest acceleration has been reached. The threshold value yi

(and also $y_2$) may here depend on, for example, vehicle speed, radius of curvature, arc angle etc. If for example the vehicle is travelling at high speed, yi is a lower value than when it is travelling at a lower speed. If $|a_{lat}|$ is greater than yi, the method moves on to step S32 and $|a_{lat}|$ is then compared with a further acceleration threshold value $y_2$. The calculation unit is adapted to measuring the period of time during which $|a_{lat}|$ exceeds $y_2$, a period here referred to as $t_{jerk}$. If $|a_{lat}|$ is greater than $y_2$ and $t_{jerk}$ is shorter than a predetermined period $t_{maxjerk}$, a grading signal which indicates a low grading is generated at step S33 on the basis of the comparison. The driver is then regarded as having for some reason made a large turn, i.e. a large steering wheel movement. For example, at step S34 a hint will be displayed to the driver via the output unit to encourage him/her to take the next bend more gently. If either or both of the conditions at step S32 are not fulfilled, the driver's acceleration behaviour is assessed according to the assessment criteria for said case 2 as regards lateral acceleration, as indicated by step S35. The result is a way of detecting whether the driver makes a rapid steering wheel movement, and of assessing his/her subsequent acceleration behaviour.

According to another embodiment, the calculation unit is adapted to calculating the derivative $\partial a_{lat}/\partial t$ for the vehicle's lateral acceleration $a_{lat}$ and determining the absolute value for the derivative, comparing this value with one or more threshold values and generating a grading signal based on the comparison. A high value of the derivative thus means a large jerk. The result is another way of detecting whether the driver makes a rapid steering wheel movement, and of assessing his/her subsequent acceleration behaviour.

According to a further embodiment, the calculation unit is adapted to determining the absolute amount for the vehicle's maximum lateral acceleration $a_{lat\_max}$ and mean lateral acceleration $a_{lat-mean}$ during a period of time t and determining whether the difference between $|a_{lat-max}|$ and $|a_{lat-mean}|$ is greater than a threshold value, and generating a grading signal based on the comparison. This embodiment is illustrated in the diagram in Figure 5, which shows the absolute amount of the vehicle's maximum and mean lateral acceleration during a period from ti to $t_2$ This difference between $|a_{lat-max}|$ and $|a_{lat-mean}|$ is illustrated as $a_{diff}$, and if it is greater than a threshold value a grading signal which indicates a jerky bend is generated. The result is a further way of detecting whether the driver makes a rapid steering wheel movement, and of assessing his/her subsequent acceleration behaviour.

High vehicle speed results in less tolerance for jerking and acceleration, so the threshold values in the above situations depend preferably on the situation-specific parameter of vehicle speed.

**Case 2**

To assess whether the driver has driven too fast on a bend, the calculation unit according to an embodiment is adapted to determining the vehicle's maximum lateral acceleration $a_{lat\_max}$ and comparing it with two threshold values $a_{th1}$ and $a_{th2}$ for the vehicle's maximum lateral acceleration, and generating a grading signal based on the comparison. The comparison is done according to an embodiment by using the formula:

$$g = \left(1 - \frac{a_{max} - a_{th1}}{a_{th2} - a_{th1}}\right) \cdot 10, \ g \in [0,...,10] \tag{1}$$

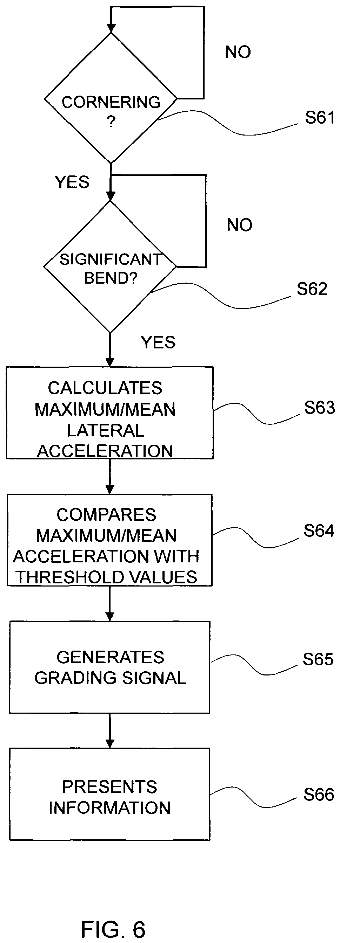

The grading signal then indicates g, which in this example is a value between 0 and 10. 10 is the maximum score and 0 the lowest possible. The flowchart in Figure 6 illustrates a method for assessing whether the driver drove too fast on a bend. According to an embodiment, a first step S61 checks whether the vehicle is cornering, e.g. if its acceleration is greater than 0. If such is the case, this is followed according to an embodiment by checking at step S62 that the bend is significant enough, e.g. long and/or sharp enough. If such is the case, an assessment may be done and the vehicle's maximum lateral acceleration $a_{lat\_max}$ be determined. Step S63 compares $a_{lat\_max}$ with the threshold values according to formula (1), and at step S64 a grading signal is generated. The information in the grading signal is then presented at step S65 via the output unit. In the event of a low grading, there is also the possibility of the driver being given a hint via the output unit to encourage him/her to reduce speed before the bend next time. According to an embodiment, the threshold values $a_{th1}$ and $a_{th2}$ are the same as the threshold values $y_1$ and $y_2$ in Figure 4.

However, assessment according to case 2 is not necessarily tied to the vehicle's maximum acceleration. Instead of using the vehicle's maximum lateral acceleration $a_{lat\_max}$ as in the above embodiment, what it used instead according to an embodiment is the vehicle's mean lateral acceleration $a_{lat\_mean}$ through the bend. In this case the calculation unit is adapted to determining the vehicle's mean lateral acceleration $a_{lat\_mean}$ and comparing it with two threshold values $a_{th3}$ and $a_{th4}$ for the vehicle's mean lateral acceleration, and to generating a grading signal based on the comparison. The flowchart in Figure 6 is therefore the same for this embodiment except that step S63 and step S64 use instead the vehicle's mean lateral acceleration, as also indicated in the flowchart.

According to an embodiment, the road ahead is known, in which case the calculation unit may be adapted to generating hints to the driver which are displayed via the input unit if he/she is about to enter a curve at too high a speed in reference to highest score.

On slippery road surfaces the vehicle may lose grip in the longitudinal direction. According to an embodiment the system takes account of factors such as slippery road surface or rain and adapts the threshold values accordingly. Hints may then also be given to the driver before or after the bend.

Various hints and forms of feedback may be given to the driver via the input unit. There follow a number of examples of conceivable feedback to the driver in an identified situation.

Where a situation with very high lateral acceleration is detected, e.g. if g=1 according to formula (1), the driver is urged to maintain a lower cornering speed. In the case of medium to high lateral acceleration, e.g. g=5 according to formula (1), if the driver braked the vehicle on the bend, he/she is urged to maintain a lower cornering speed.

In the case of low lateral acceleration, e.g. g=9 according to formula (1), if the driver released the accelerator pedal in good time before the bend and no braking occurred on the bend, he/she is praised for good driving.

In a situation with high lateral acceleration where the ratio between maximum lateral acceleration and mean lateral acceleration exceeds a threshold value, the driver is urged to avoid "jerking" during a bend.

According to an embodiment, said acceleration is the vehicle's longitudinal acceleration $a_{long}$. This makes it possible to assess how well the driver accelerates in the longitudinal direction.

Assessment of acceleration in the longitudinal direction requires according to an embodiment the fulfilment of at least one situation criterion. If at least one situation criterion is fulfilled, the calculation unit is then adapted to allowing the vehicle's longitudinal acceleration to be assessed. According to an embodiment, said situation criterion is either a longitudinal acceleration $a_{long}$ greater than a predetermined threshold value or a vehicle speed increase by more than a defined number of km/h. This makes it possible to ensure that very small changes in the vehicle's acceleration are not assessed, and also that the driver is driving in normal running conditions.

As in case 2 above, it is also possible to assess the acceleration in the longitudinal direction, referred to here as case 3.

**Case 3**

Assessment here is based on how the driver accelerates on the basis of a permissible reference acceleration which depends on the vehicle's speed. The higher the speed maintained by the vehicle, the less the driver is expected to need to accelerate. According to an embodiment, the calculation unit is adapted to determining reference values $a_{max\_long1}$ and $a_{max\_long2}$ based on threshold values for the vehicle's permissible instantaneous acceleration $a_{long}$ at current vehicle speed and comparing the vehicle's instantaneous acceleration $a_{long}$ with $a_{max\_long1}$ and $a_{max\_long2}$, and generating a grading signal based on the comparison.

The comparison is done according to an embodiment with the formula:

$$g = \left(1 - \frac{a_{long} - a_{\max\_long2}}{a_{\max\_long1} - a_{\max\_long2}}\right) \cdot 10, \, g \in [0,...10], \, a_{\max\_long1} > a_{\max\_long2}, \, a_{long} > 0 \qquad (2)$$

The grading signal then indicates the assessment of g. Formula (2) is based on instantaneous acceleration or maximum acceleration. This may of course be converted to being calculated on mean acceleration over a certain period of time, e.g. the whole or parts of the acceleration process. The calculation unit is then adapted to determining reference values $a_{mean\_long1}$ and $a_{mean\_long2}$ based on threshold values for the vehicle's permissible mean acceleration $a_{mean\_long}$ at current mean speed and comparing its mean acceleration $a_{mean\_long}$ with $a_{mean\_long1}$ and $a_{mean\_long2}$, and generating a grading signal based on the comparison. Formula (2) may be used for comparison, but with changed parameters.

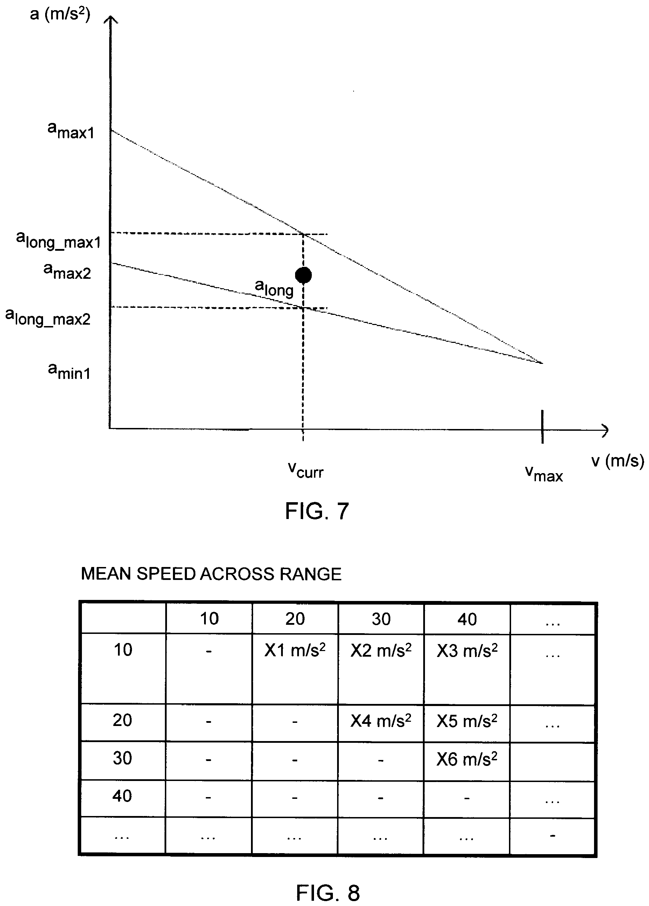

Figure 7 is a diagram illustrating how the reference values $a_{max\_long1}$ and $a_{max\_long2}$ and the reference values $a_{mean\_long1}$ and $a_{mean\_long2}$ are calculated for the instantaneous acceleration $a_{long}$. The y-axis denotes acceleration values, the x-axis speed values. The following are given: the vehicle's longitudinal acceleration $a_{long}$, current speed $V_{curr}$ and maximum speed $V_{max}$, a minimum acceleration amin and two threshold values $a_{max1}$ and $a_{max2}$ for the vehicle's permissible reference acceleration. From these it is possible to arrive at limits for the vehicle's permissible longitudinal acceleration. Depending on the values of $a_{long}$ and $V_{curr}$, the reference values are then arrived at. The diagram illustrates clearly that the higher the vehicle's speed, the smaller the permissible acceleration.

Another embodiment uses a table of speed ranges and permissible acceleration levels to assess the driver's acceleration behaviour. An example of such a table appears in Figure 8. If for example the vehicle's speed changes from 10 km/h to 20 km/h, the permissible acceleration according to the table is X1 m/s$^2$. The calculation unit in this embodiment is adapted to monitoring the vehicle's speed and acceleration, and when an increase in its speed within any of the ranges in the diagram is observed, its acceleration is compared with the permissible acceleration in the table, and a grading signal is generated. If for example its acceleration is greater than that in the table, the grading signal will indicate a low score. According to an embodiment, the table values X1-Xn are variable and depend for example on the vehicle's weight. For example, higher accelerations are permissible at low vehicle weight.

Said acceleration a comprises according to an embodiment both lateral and longitudinal acceleration, which makes it possible to assess both the lateral and the longitudinal acceleration as described above.

The invention relates also to a computer programme product which comprises computer programme instructions to enable a computer system in a vehicle to perform the steps according to the method when the computer programme instructions are run on said computer system. The invention comprises also a computer programme product which has the computer programme instructions stored on a medium which can be read by a computer system.

The present invention is not limited to the embodiments described above. Various alternatives, modifications or equivalents may be used. The aforesaid embodiments therefore do not limit the scope of the invention, which is defined by the attached claims.

**Claims**

1. An assessment method for assessing acceleration behaviour of a driver of a vehicle, **characterised in that** the method comprises:

    - determining (S21) the vehicle's acceleration (a) being lateral and/or longitudinal acceleration;
    - calculating (S22) one or more threshold values for its acceleration which depend on at least one situation-specific parameter, wherein said one or more threshold values indicate limits for what is regarded as safe and comfortable lateral acceleration or safe and comfortable longitudinal acceleration;
    - comparing (S23) the vehicle's acceleration (a) with said one or more threshold values, and generating (S24) on the basis of the comparison a grading signal related to said calculated one or more threshold values, with presentation of information from the grading signal to the driver after said acceleration a has ended, said situation-specific parameter being any from among the vehicle's speed, its acceleration, the radius of curvature of the road or the arc angle of the road, in which said acceleration is the vehicle's lateral acceleration ($a_{lat}$) and wherein said method comprises doing an assessment of the vehicle's lateral acceleration if at least one situation criterion is fulfilled, wherein said situation criterion is any from among a lowest vehicle speed, a lowest acceleration, a largest radius of curvature or a smallest arc angle.

2. An assessment method according to claim 1, which comprises determining the absolute amount for ($a_{lat}$) during a predetermined period of time ($t_{maxjerk}$) and comparing this value with a threshold value ($y_2$) which represents said one or more threshold values, and generating a grading signal based on the comparison.

3. An assessment method according to claim 1, which comprises calculating the derivative ($\partial a_{lat}/\partial t$) for the vehicle's lateral acceleration ($a_{lat}$) and determining the absolute amount for the derivative, comparing this value with said one or more threshold values and generating a grading signal based on the comparison.

4. An assessment method according to claim 1, which comprises determining the absolute amount for the vehicle's maximum lateral acceleration ($a_{lat\_max}$) and mean lateral acceleration ($a_{lat\_mean}$) during a period of time (t) and determining whether the difference between $|a_{lat\_max}|$ and $|a_{lat\_mean}|$ is greater than said one or more threshold values, and generating a grading signal based on the comparison.

5. An assessment method according to claim 1, which comprises determining the vehicle's maximum lateral acceleration ($a_{lat\_max}$) and comparing it with two threshold values ($a_{th1}$) and (ath2) for the vehicle's maximum lateral acceleration which represent said one or more threshold values, and generating a grading signal based on the comparison.

6. An assessment method according to claim 1, which comprises determining the vehicle's mean lateral acceleration ($a_{lat\_mean}$) and comparing it with two threshold values (ath3) and (ath4) for the vehicle's mean lateral acceleration which represent said one or more threshold values, and generating a grading signal based on the comparison.

7. An assessment method according to claim 1, in which said acceleration is the vehicle's longitudinal acceleration (along).

8. An assessment method according to claim 7, which comprises doing an assessment of the vehicle's longitudinal acceleration if ($a_{long}$) is greater than a predetermined threshold value and/or the vehicle's speed increases by more than a defined number of km/h.

9. An assessment method according to claim 7 or 8, which comprises determining reference values ($a_{max\_long1}$) and ($a_{max\_long2}$) based on threshold values for the vehicle's permissible instantaneous acceleration ($a_{long}$) at current vehicle speed which represent said one or more threshold values and comparing the vehicle's instantaneous acceleration ($a_{long}$) with ($a_{max\_long1}$ and ($a_{max\_long2}$), and generating a grading signal based on the comparison.

10. An assessment method according to claim 7 or 8, which comprises determining reference values ($a_{mean\_long1}$) and ($a_{mean\_long2}$) based on threshold values for the vehicle's permissible mean acceleration ($a_{mean\_long}$) at current mean speed which represent said one or more threshold values, and comparing the vehicle's mean acceleration ($a_{mean\_long}$) with ($a_{mean\_long1}$) and ($a_{mean\_long2}$), and generating a grading signal based on the comparison.

11. An assessment method according to any one of the foregoing claims, which comprises, at the time when a grading signal is generated, feeding back to the driver via an output unit how he/she can improve his/her acceleration behaviour, or praising the driver's acceleration behaviour by his/her receiving information from the grading signal.

12. An assessment system for assessing acceleration behaviour of a driver of a vehicle, which system comprises at least one sensor unit adapted to delivering an acceleration signal which indicates the vehicle's acceleration (a) being lateral and/or longitudinal acceleration,
**characterised in that** the system comprises

- a calculation unit adapted to receiving said acceleration signal, calculating one or more threshold values for the vehicle's acceleration which depend on at least one situation-specific parameter, wherein said one or more threshold values indicate limits for what is regarded as safe and comfortable lateral acceleration or safe and comfortable longitudinal acceleration, comparing its acceleration a with said one or more threshold values, and generating on the basis of the comparison a grading signal related to said calculated one or more threshold values, and
- an output unit adapted to presenting information from the grading signal to the driver after said acceleration has ended, said situation-specific parameter being any from among the vehicle's speed, its acceleration, the radius of curvature of the road or the arc angle of the road, in which said acceleration is the vehicle's lateral acceleration ($a_{lat}$) and wherein the calculation unit is adapted to determining whether at least one situation criterion is fulfilled and therefore allowing an assessment of the vehicle's lateral acceleration to be done, wherein said situation criterion is any from among a lowest vehicle speed, a largest radius of curvature or a smallest arc angle.

13. An assessment system according to claim 12, in which the calculation unit is adapted to determining the absolute amount for ($a_{lat}$) during a predetermined period of time ($t_{maxjerk}$) and comparing this value with a threshold value ($y_2$) which represents said one or more threshold values, and generating a grading signal based on the comparison.

14. An assessment system according to claim 12, in which the calculation unit is adapted to calculating the derivative ($\partial a_{lat}/\partial t$) for the vehicle's lateral acceleration ($a_{lat}$) and determining the absolute amount for the derivative, comparing this value with said one or more threshold values and generating a grading signal based on the comparison.

15. An assessment system according to claim 12, in which the calculation unit is adapted to determining the absolute amount for the vehicle's maximum lateral acceleration ($a_{lat\_max}$) and mean lateral acceleration ($a_{lat\_mean}$) during a period of time (t) and determining whether the difference between $|a_{lat\_max}|$ and $|a_{lat\_mean}|$ is greater than said one or more threshold values, and generating a grading signal based on the comparison.

16. An assessment system according to claim 12, in which the calculation unit is adapted to determining the vehicle's maximum lateral acceleration ($a_{lat\_max}$) and comparing it with two threshold values ($a_{th1}$) and ($a_{th2}$) for the vehicle's maximum lateral acceleration which represent said one or more threshold values, and generating a grading signal based on the comparison.

17. An assessment system according to claim 12, in which the calculation unit is adapted to determining the vehicle's mean lateral acceleration ($a_{lat\_mean}$) and comparing it with two threshold values (ath3) and (ath4) for the vehicle's mean lateral acceleration which represent said one or more threshold values, and generating a grading signal based on the comparison.

18. An assessment system according to claim 12, in which said acceleration is the vehicle's longitudinal acceleration ($a_{long}$).

**19.** An assessment system according to claim 18, in which the calculation unit is adapted to determining whether ($a_{long}$) is greater than a predetermined threshold value and/or whether the vehicle's speed increases by more than a defined number of km/h, in which case it allows an assessment of its longitudinal acceleration to be done.

**20.** An assessment system according to claim 18 or 19, in which the calculation unit is adapted to determining reference values ($a_{max\_long1}$) and ($a_{max\_long2}$) based on threshold values for the vehicle's permissible instantaneous acceleration ($a_{long}$) at current vehicle speed which represent said one or more threshold values and comparing the vehicle's instantaneous acceleration ($a_{long}$) with ($a_{max\_long1}$) and ($a_{max\_long2}$), and generating a grading signal based on the comparison.

**21.** An assessment system according to claim 18 or 19, in which the calculation unit is adapted to determining reference values ($a_{mean\_long1}$) and ($a_{mean\_long2}$) based on threshold values for the vehicle's permissible mean acceleration ($a_{mean\_long}$) at current mean speed which represent said one or more threshold values, and comparing its mean acceleration ($a_{mean\_long}$) with ($a_{mean\_long1}$) and ($a_{mean\_long2}$), and generating a grading signal based on the comparison.

**22.** An assessment system according to any one of claims 12 to 21, in which the calculation unit is adapted, at the time when a grading signal is generated, to feeding back to the driver via an output unit how he/she can improve his/her acceleration behaviour, or to praising the driver's acceleration behaviour by his/her receiving information from the grading signal.

**23.** A computer programme product comprising computer programme instructions for enabling a computer system in a vehicle to perform steps according to the method according to any of claims 1 to 11 when the computer programme instructions are run on said computer system.

**24.** A computer programme product according to claim 23, which has the computer programme instructions stored on a medium which can be read by a computer system.

**Patentansprüche**

**1.** Bestimmungsverfahren zum Bestimmen eines Beschleunigungsverhaltens eines Fahrers eines Fahrzeugs, **dadurch gekennzeichnet, dass** das Verfahren umfasst:

- Ermitteln (S21), ob die Beschleunigung (a) des Fahrzeugs eine Quer- und/oder Längsbeschleunigung ist,
- Berechnen (S22) eines oder mehrerer Schwellenwerte für seine Beschleunigung, die von zumindest einem situationsspezifischen Parameter abhängig sind, wobei der eine oder die mehreren Schwellenwerte Grenzen für das angeben, was als eine sichere und komfortable Querbeschleunigung oder eine sichere und komfortable Längsbeschleunigung angesehen wird,
- Vergleichen (S23) der Fahrzeugbeschleunigung (a) mit dem einen oder den mehreren Schwellenwerten und, basierend auf dem Vergleich, Erzeugen (S24) eines mit dem einen oder den mehreren berechneten Schwellenwerten in Beziehung stehenden Klassifizierungssignals mit einer Präsentation von Information aus dem Klassifizierungssignal an den Fahrer nachdem die Beschleunigung beendet ist, wobei der situationsspezifische Parameter ein jeglicher aus der Geschwindigkeit des Fahrzeugs, seiner Beschleunigung, dem Krümmungsradius der Straße oder dem Bogenradius der Straße ist, wobei die Beschleunigung die Querbeschleunigung ($a_{lat}$) des Fahrzeugs ist und wobei das Verfahren umfasst eine Durchführung einer Bestimmung der Fahrzeugquerbeschleunigung, falls zumindest ein Situationskriterium erfüllt ist, wobei das Situationskriterium ein jegliches aus einer geringsten Fahrzeuggeschwindigkeit, einer geringsten Beschleunigung, einem größten Krümmungsradius oder einem kleinsten Bogenradius ist.

**2.** Bestimmungsverfahren nach Anspruch 1, welches ein Bestimmen des Absolutbetrages für ($a_{lat}$) während einer vorbestimmten Zeitdauer ($t_{maxjerk}$) und ein Vergleichen dieses Wertes mit einem Schwellenwert ($y_2$), der den einen oder die mehreren Schwellenwerte darstellt, und ein Erzeugen eines Klassifizierungssignals basierend auf dem Vergleich umfasst.

**3.** Bestimmungsverfahren nach Anspruch 1, welches ein Berechnen der Ableitung ($\delta a_{lat}/\delta_t$) für die Fahrzeugquerbeschleunigung ($a_{lat}$) und ein Bestimmen des Absolutbetrages für die Ableitung, ein Vergleichen dieses Wertes mit dem einen oder den mehreren Schwellenwerten und ein Erzeugen eines Klassifizierungssignals basierend auf dem Vergleich umfasst.

4. Bestimmungsverfahren nach Anspruch 1, welches ein Bestimmen des Absolutbetrags für die maximale Fahrzeugquerbestimmung ($a_{lat\_max}$) und mittlere Querbeschleunigung ($a_{lat\_mean}$) während einer Zeitdauer (t) und ein Feststellen, ob die Differenz zwischen ($a_{lat\_max}$) und ($a_{lat\_mean}$) größer ist als der eine oder die mehreren Schwellenwerte, und ein Erzeugen eines Klassifizierungssignals basierend auf dem Vergleich umfasst.

5. Bestimmungsverfahren nach Anspruch 1, welches ein Bestimmen der maximalen Fahrzeugquerbeschleunigung ($a_{lat\_max}$) und ein Vergleichen derselben mit zwei Schwellenwerten ($a_{th1}$) und ($a_{th2}$) für die maximale Fahrzeugquerbeschleunigung, die den einen oder die mehreren Schwellenwerte darstellen, und ein Erzeugen eines Klassifizierungssignals basierend auf dem Vergleich umfasst.

6. Bestimmungsverfahren nach Anspruch 1, welches ein Bestimmen der mittleren Fahrzeugquerbeschleunigung ($a_{lat\_mean}$) und Vergleichen derselben mit zwei Schwellenwerten ($a_{th3}$) und ($a_{th4}$) für die mittlere Fahrzeugquerbeschleunigung, die den einen oder die mehreren Schwellenwerte darstellen, und ein Erzeugen eines Klassifizierungssignals basierend auf dem Vergleich umfasst.

7. Bestimmungsverfahren nach Anspruch 1, bei dem die Beschleunigung die Längsbeschleunigung ($a_{long}$) des Fahrzeugs ist.

8. Bestimmungsverfahren nach Anspruch 7, welches ein Durchführen einer Bestimmung der Fahrzeuglängsbeschleunigung umfasst, falls ($a_{long}$) größer ist als ein vorbestimmter Schwellenwert und/oder die Fahrzeuggeschwindigkeit um mehr als eine definierte Anzahl von km/h ansteigt.

9. Bestimmungsverfahren nach Anspruch 7 oder 8, welches ein Bestimmen von Referenzwerten ($a_{max\_long1}$) und ($a_{max\_long2}$) basierend auf Schwellenwerten für die zulässige unmittelbare Fahrzeugbeschleunigung ($a_{long}$) bei momentaner Fahrzeuggeschwindigkeit, welche den einen oder die mehreren Schwellenwerte darstellen, und ein Vergleichen der unmittelbaren Fahrzeugbeschleunigung ($a_{long}$) mit ($a_{max\_long1}$) und ($a_{max\_long2}$) sowie ein Erzeugen eines Klassifizierungssignals basierend auf dem Vergleich umfasst.

10. Bestimmungsverfahren nach Anspruch 7 oder 8, welches ein Bestimmen von Referenzwerten ($a_{mean\_long1}$) und ($a_{mean\_long2}$) basierend auf Schwellenwerten für die zulässige mittlere Fahrzeugbeschleunigung ($a_{mean\_long}$) bei momentaner mittlerer Geschwindigkeit, die den einen oder die mehreren Schwellenwerte darstellen, und ein Vergleichen der mittleren Fahrzeugbeschleunigung ($a_{mean\_long}$) mit ($a_{mean\_long1}$) und ($a_{mean\_long2}$) sowie ein Erzeugen eines Klassifizierungssignals basierend auf dem Vergleich umfasst.

11. Bestimmungsverfahren nach einem der vorhergehenden Ansprüche, welches eine Rückmeldung, zum Zeitpunkt des Erzeugens eines Klassifizierungssignals, an den Fahrer über eine Ausgabeeinheit, wie er/sie sein/ihr Beschleunigungsverhalten verbessern kann, oder ein Loben des Beschleunigungsverhaltens des Fahrers dadurch umfasst, dass er/sie Information aus dem Klassifizierungssignal erhalten.

12. Bestimmungssystem zum Bestimmen eines Beschleunigungsverhaltens eines Fahrers eines Fahrzeugs, wobei das System wenigstens eine zum Liefern eines Beschleunigungssignals ausgeführte Sensoreinheit aufweist, die angibt, ob die Fahrzeugbeschleunigung (a) eine Quer- und/oder Längsbeschleunigung ist,
   **dadurch gekennzeichnet, dass** das System aufweist

   - eine Berechnungseinheit, die eingerichtet ist zum Empfangen des Beschleunigungssignals, Berechnen eines oder mehrerer Schwellenwerte für die Fahrzeugbeschleunigung, die von wenigstens einem situationsspezifischen Parameter abhängig sind, wobei der eine oder die mehreren Schwellenwerte Grenzen für das angeben, was als eine sichere und komfortable Querbeschleunigung oder eine sichere und komfortable Längsbeschleunigung angesehen wird, Vergleichen seiner Beschleunigung (a) mit dem einen oder den mehreren Schwellenwerten, und Erzeugen, basierend auf dem Vergleich, eines mit dem einen oder den mehreren berechneten Schwellenwerten in Beziehung stehenden Klassifizierungssignals, und
   - eine Ausgabeeinheit, die dazu eingerichtet ist, Information aus dem Klassifizierungssignal dem Fahrer zu präsentieren, nachdem die Beschleunigung beendet ist, wobei der situationsspezifische Parameter ein jeglicher aus der Geschwindigkeit des Fahrzeugs, seiner Beschleunigung, dem Krümmungsradius der Straße oder dem Bogenradius der Straße ist, wobei die Beschleunigung die Fahrzeugquerbeschleunigung ($a_{lat}$) ist und wobei die Berechnungseinheit dazu eingerichtet ist festzustellen, ob zumindest ein Situationskriterium erfüllt ist und daher ein Durchführen einer Bestimmung der Fahrzeugquerbeschleunigung gestattet, wobei das Situationskriterium ein jegliches aus einer geringsten Fahrzeuggeschwindigkeit, einem größten Krümmungsradius oder einem kleinsten Bogenradius ist.

13. Bestimmungssystem nach Anspruch 12, bei dem die Berechnungseinheit dazu eingerichtet ist, den Absolutbetrag für ($a_{lat}$) während einer vorbestimmten Zeitdauer ($t_{maxjerk}$) zu ermitteln und diesen Wert mit einem Schwellenwert ($y_2$) zu vergleichen, der den einen oder die mehreren Schwellenwerte darstellt, und basierend auf dem Vergleich ein Klassifizierungssignal zu erzeugen.

14. Bestimmungssystem nach Anspruch 12, bei dem die Berechnungseinheit dazu eingerichtet ist, die Ableitung ($\delta a_{lat}/\delta_t$) für die Fahrzeugquerbeschleunigung ($a_{lat}$) zu berechnen und den Absolutbetrag für die Ableitung zu bestimmen, diesen Wert mit dem einen oder den mehreren Schwellenwerten zu vergleichen und basierend auf dem Vergleich ein Klassifizierungssignal zu erzeugen.

15. Bestimmungssystem nach Anspruch 12, bei dem die Berechnungseinheit dazu eingerichtet ist, den Absolutbetrag der maximalen Fahrzeugquerbeschleunigung ($a_{lat\_max}$) und mittleren Querbeschleunigung ($a_{lat\_mean}$) während einer Zeitdauer (t) zu bestimmen und festzustellen, ob die Differenz zwischen ($a_{lat\_max}$) und ($a_{lat\_mean}$) größer ist als der eine oder die mehreren Schwellenwerte, und basierend auf dem Vergleich ein Klassifizierungssignal zu erzeugen.

16. Bestimmungssystem nach Anspruch 12, bei dem die Berechnungseinheit dazu eingerichtet ist, die maximale Fahrzeugquerbeschleunigung ($a_{lat\_max}$) zu bestimmen und sie mit zwei Schwellenwerten ($a_{th1}$) und (ath2) für die maximale Fahrzeugquerbeschleunigung, welche den einen oder die mehreren Schwellenwerte darstellen, zu vergleichen und basierend auf dem Vergleich ein Klassifizierungssignal zu erzeugen.

17. Bestimmungssystem nach Anspruch 12, bei dem die Berechnungseinheit dazu eingerichtet ist, die mittlere Fahrzeugquerbeschleunigung ($a_{lat\_mean}$) zu bestimmen und sie mit zwei Schwellenwerten (ath3) und (ath4) für die mittlere Fahrzeugquerbeschleunigung, welche den einen oder die mehreren Schwellenwerte darstellen, zu vergleichen und basierend auf dem Vergleich ein Klassifizierungssignal zu erzeugen.

18. Bestimmungssystem nach Anspruch 12, bei dem die Beschleunigung die Fahrzeuglängsbeschleunigung ($a_{long}$) ist.

19. Bestimmungssystem nach Anspruch 18, bei dem die Berechnungseinheit dazu eingerichtet ist zu bestimmen, ob ($a_{long}$) größer ist als ein vorbestimmter Schwellenwert und/oder ob die Fahrzeuggeschwindigkeit um mehr als eine festgelegte Anzahl von km/h zunimmt, zutreffendenfalls sie ein Durchführen einer Bestimmung seiner Längsbeschleunigung gestattet.

20. Bestimmungssystem nach Anspruch 18 oder 19, bei dem die Berechnungseinheit dazu eingerichtet ist, Referenzwerte ($a_{max-long1}$) und ($a_{max-long2}$) basierend auf Schwellenwerten für die zulässige unmittelbare Fahrzeugbeschleunigung ($a_{long}$) bei momentaner Fahrzeuggeschwindigkeit, welche den einen oder die mehreren Schwellenwerte darstellen, zu bestimmen und die unmittelbare Fahrzeugbeschleunigung ($a_{long}$) mit ($a_{max\_long1}$) und ($a_{max\_long2}$) zu vergleichen und basierend auf dem Vergleich ein Klassifizierungssignal zu erzeugen.

21. Bestimmungssystem nach Anspruch 18 oder 19, bei dem die Berechnungseinheit dazu eingerichtet ist, Referenzwerte ($a_{mean\_long1}$) und ($a_{mean\_long2}$) basierend auf Schwellenwerten für die zulässige mittlere Fahrzeugbeschleunigung ($a_{mean\_long}$) bei momentaner mittlerer Geschwindigkeit, welche den einen oder die mehreren Schwellenwerte darstellen, zu bestimmen und seine mittlere Beschleunigung ($a_{mean\_long}$) mit ($a_{mean\_long1}$) und ($a_{mean\_long2}$) zu vergleichen und basierend auf dem Vergleich ein Klassifizierungssignal zu erzeugen.

22. Bestimmungssystem nach einem der Ansprüche 12 bis 21, bei dem die Berechnungseinheit dazu eingerichtet ist, zum Zeitpunkt des Erzeugens eines Klassifizierungssignals, dem Fahrer über eine Ausgabeeinheit eine Rückmeldung zu geben, wie er/sie sein/ihr Beschleunigungsverhalten verbessern kann oder das Beschleunigungsverhalten des Fahrers zu loben, indem er/sie Information aus dem Klassifizierungssignal empfängt.

23. Computerprogrammprodukt mit Computerprogrammanweisungen zum Ermöglichen, dass ein Computersystem in einem Fahrzeug Schritte gemäß dem Verfahren nach einem der Ansprüche 1 bis 11 ausführt, wenn die Computerprogrammanweisungen auf dem Computersystem ablaufen.

24. Computerprogrammprodukt nach Anspruch 23, das die Computerprogrammanweisungen auf einem Medium gespeichert hat, das von einem Computersystem gelesen werden kann.

**Revendications**

1. Procédé d'évaluation pour évaluer le comportement d'accélération d'un conducteur d'un véhicule, **caractérisé en ce que** le procédé comprend :

   - la détermination (S21) de l'accélération du véhicule (a) étant une accélération latérale et/ou longitudinale ;
   - le calcul (S22) d'une ou de plusieurs valeurs seuils pour son accélération qui dépendent d'au moins un paramètre spécifique à la situation, dans lequel ladite ou plusieurs valeurs de seuil indiquent des limites pour ce qui est considéré comme une accélération latérale sûre et confortable ou une accélération longitudinale sûre et confortable ;
   - la comparaison (S23) de l'accélération du véhicule (a) avec ladite ou plusieurs valeurs seuil, et la génération (S24) sur la base de la comparaison du signal de classification lié à ladite valeur calculée d'une ou de plusieurs valeurs seuil, avec la présentation d'informations du signal de classification au conducteur après que ladite accélération est terminée, ledit paramètre spécifique à la situation étant un parmi la vitesse du véhicule, son accélération, le rayon de courbure de la route ou l'angle de l'arc de la route, dans lequel ladite accélération est l'accélération latérale du véhicule ($a_{lat}$) et dans lequel ledit procédé comprend la réalisation d'une évaluation de l'accélération latérale du véhicule si au moins un critère de situation est satisfait, dans lequel ledit critère de situation figure parmi une vitesse de véhicule la plus faible, une accélération plus faible, un rayon de courbure plus important ou un angle d'arc plus petit.

2. Procédé d'évaluation selon la revendication 1, qui comprend la détermination du montant absolu pour ($a_{lat}$) pendant une période de temps prédéterminée ($t_{maxjerk}$) et la comparaison de cette valeur à une valeur seuil ($y_2$) qui représente ladite ou plusieurs valeurs seuils, et la génération d'un signal de classification basé sur la comparaison.

3. Procédé d'évaluation selon la revendication 1, qui comprend le calcul du dérivé ($\partial_{ala}/\delta_t$) pour l'accélération latérale du véhicule ($a_{lat}$) et la détermination du montant absolu pour le dérivé, la comparaison de cette valeur avec ladite ou plusieurs valeurs seuils et la génération d'un signal de classification basé sur la comparaison.

4. Procédé d'évaluation selon la revendication 1, qui comprend la détermination du montant absolu pour l'accélération latérale maximale du véhicule (aiat_max) et une accélération latérale moyenne ($a_{lat\_moy}$) pendant une période de temps (t) et la détermination si la différence entre $|a_{lat\_max}|$ et $|a_{lat\_moy}|$ est supérieure à ladite ou plusieurs valeurs seuils et la génération d'un signal de classification basé sur la comparaison.

5. Procédé d'évaluation selon la revendication 1, qui comprend la détermination de l'accélération latérale maximale du véhicule ($a_{lat\_max}$) et la comparaison de celle-ci à deux valeurs seuil ($a_{th1}$) et ($a_{th2}$) pour l'accélération latérale maximale du véhicule qui représente ladite ou plusieurs valeurs seuil, et la génération d'un signal de classification basé sur la comparaison.

6. Procédé d'évaluation selon la revendication 1, qui comprend la détermination de l'accélération latérale moyenne du véhicule ($a_{lat\_moy}$) et la comparaison de celle-ci à deux valeurs seuil (ath3) et (ath4) pour l'accélération latérale moyenne du véhicule qui représente une ou plusieurs valeurs seuils, et la génération d'un signal de classification basé sur la comparaison.

7. Procédé d'évaluation selon la revendication 1, dans lequel ladite accélération est l'accélération longitudinale du véhicule ($a_{long}$) .

8. Procédé d'évaluation selon la revendication 7, qui comprend l'évaluation de l'accélération longitudinale du véhicule si ($a_{long}$) est supérieure à une valeur seuil prédéterminée et/ou si la vitesse du véhicule augmente de plus d'un nombre défini de km/h.

9. Procédé d'évaluation selon la revendication 7 ou 8, qui comprend la détermination des valeurs de référence ($a_{max\_long1}$) et ($a_{max\_long2}$) basé sur les valeurs seuil pour l'accélération instantanée autorisée du véhicule ($a_{long}$) à la vitesse actuelle du véhicule, ce qui représente ladite ou plusieurs valeurs seuil et la comparaison de l'accélération instantanée du véhicule ($a_{long}$) avec ($a_{max\_long1}$) et ($a_{max\_long2}$) et la génération d'un signal de classification basé sur la comparaison.

**10.** Procédé d'évaluation selon la revendication 7 ou 8, qui comprend la détermination des valeurs de référence ($a_{moy\_long1}$) et (amoy_long2) sur la base des valeurs seuil pour l'accélération moyenne autorisée du véhicule ($a_{moy\_long2}$) à la vitesse moyenne actuelle qui représente ladite ou plusieurs valeurs seuils, et la comparaison de l'accélération moyenne des véhicules ($a_{moy\_long}$) avec ($a_{moy\_long1}$) et (amoy_long2), et la génération d'un signal de classification basé sur la comparaison.

**11.** Procédé d'évaluation selon l'une quelconque des revendications précédentes, qui comprend, au moment où un signal de classification est généré, la retransmission au conducteur via une unité de sortie de la manière avec laquelle il/elle peut améliorer son comportement d'accélération, ou les félicitations données au comportement d'accélération du conducteur par son information de réception du signal de classification.

**12.** Système d'évaluation pour évaluer le comportement d'accélération d'un conducteur d'un véhicule, lequel système comprend au moins une unité de capteur adaptée à la fourniture d'un signal d'accélération qui indique que l'accélération du véhicule (a) est une accélération latérale et/ou longitudinale,
**caractérisé en ce que** le système comprend

- une unité de calcul adaptée à la réception dudit signal d'accélération, le calcul d'une ou de plusieurs valeurs seuil pour l'accélération du véhicule qui dépend d'au moins un paramètre spécifique à la situation, dans lequel ladite ou plusieurs valeurs seuil indiquent des limites pour ce qui est considéré comme une accélération latérale sûre et confortable ou une accélération longitudinale sûre et confortable, la comparaison de son accélération a à ladite ou plusieurs valeurs seuil, et la génération sur la base de la comparaison, d'un signal de classification lié à ladite valeur calculée ou plusieurs valeurs seuil, et
- une unité de sortie adaptée à la présentation des informations du signal de classification au conducteur après que ladite accélération a expiré, ledit paramètre spécifique à la situation étant l'un quelconque parmi la vitesse du véhicule, son accélération, le rayon de courbure de la route ou l'angle de l'arc de la route, dans lequel ladite accélération est l'accélération latérale du véhicule ($a_{lat}$) et dans lequel l'unité de calcul est adaptée pour déterminer si au moins un critère de situation est satisfait et, par conséquent, permettre une évaluation de l'accélération latérale du véhicule à réaliser, dans lequel ledit critère de situation figure parmi une vitesse de véhicule la plus faible, un rayon de courbure plus important ou un angle d'arc plus petit.

**13.** Système d'évaluation selon la revendication 12, dans lequel l'unité de calcul est adaptée à la détermination du montant absolu pour ($a_{lat}$) pendant une période de temps prédéterminée ($t_{maxjerk}$) et la comparaison de cette valeur à une valeur seuil ($y_2$) qui représente ladite ou plusieurs valeurs seuils, et la génération d'un signal de classification basé sur la comparaison.

**14.** Système d'évaluation selon la revendication 12, dans lequel l'unité de calcul est adaptée au calcul du dérivé ($\partial alat/\partial t$) pour l'accélération latérale du véhicule ($a_{lat}$) et la détermination du montant absolu pour le dérivé, la comparaison de cette valeur avec une ou plusieurs valeurs seuils et la génération d'un signal de classification basé sur la comparaison.

**15.** Système d'évaluation selon la revendication 12, dans lequel l'unité de calcul est adaptée à la détermination du montant absolu pour l'accélération latérale maximale du véhicule ($a_{lat\_max}$) et l'accélération latérale moyenne ($a_{lat\_moy}$) pendant une période de temps (t) et la détermination si la différence entre $|a_{lat\_max}|$ et $|a_{lat\_moy}|$ est supérieure à une ou plusieurs valeurs seuils et la génération d'un signal de classification basé sur la comparaison.

**16.** Système d'évaluation selon la revendication 12, dans lequel l'unité de calcul est adaptée à la détermination de l'accélération latérale maximale du véhicule ($a_{lat\_max}$) et la comparaison de celle-ci avec deux valeurs seuil ($a_{th1}$) et ($a_{th2}$) pour l'accélération latérale maximale du véhicule qui représente ladite ou plusieurs valeurs seuil, et la génération d'un signal de classification basé sur la comparaison.

**17.** Système d'évaluation selon la revendication 12, dans lequel l'unité de calcul est adaptée à la détermination de l'accélération latérale moyenne du véhicule ($a_{lat\_moy}$) et la comparaison de celle-ci avec deux valeurs seuil (ath3) et (ath4) pour l'accélération latérale moyenne du véhicule qui représente ladite ou plusieurs valeurs seuil, et la génération d'un signal de classification basé sur la comparaison.

**18.** Système d'évaluation selon la revendication 12, dans lequel ladite accélération est l'accélération longitudinale du véhicule ($a_{long}$).

EP 2 561 502 B1

**19.** Système d'évaluation selon la revendication 18, dans lequel l'unité de calcul est adaptée pour déterminer si ($a_{long}$) est supérieure à une valeur seuil prédéterminée et/ou si la vitesse du véhicule augmente de plus d'un nombre défini de km/h, auquel cas il permet de réaliser une évaluation de son accélération longitudinale à réaliser.

**20.** Système d'évaluation selon la revendication 18 ou 19, dans lequel l'unité de calcul est adaptée à la détermination des valeurs de référence ($a_{max\_long1}$) et ($a_{max\_long2}$) basé sur les valeurs seuil pour l'accélération instantanée autorisée du véhicule ($a_{long}$) à la vitesse actuelle du véhicule, ce qui représente une ou plusieurs valeurs seuil et la comparaison de l'accélération instantanée du véhicule ($a_{long}$) avec ($a_{max\_long1}$) et ($a_{max\_long2}$) et la génération d'un signal de classification basé sur la comparaison.

**21.** Système d'évaluation selon la revendication 18 ou 19, dans lequel l'unité de calcul est adaptée à la détermination des valeurs de référence ($a_{moy\_long1}$) et ($a_{moy-long2}$) basé sur les valeurs seuil pour l'accélération moyenne autorisée du véhicule ($a_{moy\_long}$) à la vitesse moyenne actuelle qui représente ladite ou plusieurs valeurs seuils, et la comparaison de son accélération moyenne ($a_{moy\_long}$) avec ($a_{moy\_long1}$) et ($a_{moy-long2}$ ) et la génération d'un signal de classification basé sur la comparaison.

**22.** Système d'évaluation selon l'une quelconque des revendications 12 à 21, dans lequel l'unité de calcul est adaptée, au moment où un signal de classification est généré, à la retransmission au conducteur via une unité de sortie de la manière avec laquelle il/elle peut améliorer son comportement d'accélération, ou les félicitations pour le comportement d'accélération du conducteur par son information de réception du signal de classification.

**23.** Produit de programme informatique comprenant des instructions de programme informatique pour permettre à un système informatique dans un véhicule d'exécuter les étapes selon le procédé selon l'une quelconque des revendications 1 à 11, lorsque les instructions de programme informatique sont exécutées sur ledit système informatique.

**24.** Produit de programme informatique selon la revendication 23, dans lequel les instructions de programme informatique stockées sur un support peuvent être lues par un système informatique.

14

ASSESSMENT SYSTEM

| SENSOR UNIT 1 |

ACCELERATION
SIGNAL/SIGNALS

| SENSOR UNIT n |

CALCULATION UNIT

| PROCESSOR UNIT |

| MEMORY |

| OUTPUT UNIT |

GRADING SIGNAL

FIG. 1

FIG. 2

NO

ABS $(a_{lat}) > y_1$?

S31

YES

ASSESS ACCORDING TO NORMAL CURVE

S35

NO

ABS $(a_{lat}) > y_2$ and $t_{jerk} < t_{jerkmax}$?

S32

YES

GENERATES GRADING SIGNAL

S33

GIVES HINTS

S34

FIG. 3

FIG. 4

FIG. 5

```
          ┌──────────┐
          │          │
          ▼          │  NO
        ◇ CORNERING ◇──────────── S61
        ◇     ?     ◇
          │
     YES  │          ┌──────────┐
          ▼          │          │  NO
        ◇ SIGNIFICANT ◇──────── S62
        ◇   BEND?    ◇
          │
     YES  │
          ▼
   ┌─────────────────┐
   │   CALCULATES    │
   │  MAXIMUM/MEAN   │──── S63
   │    LATERAL      │
   │  ACCELERATION   │
   └─────────────────┘
          │
          ▼
   ┌─────────────────┐
   │   COMPARES      │
   │  MAXIMUM/MEAN   │──── S64
   │ ACCELERATION WITH│
   │ THRESHOLD VALUES│
   └─────────────────┘
          │
          ▼
   ┌─────────────────┐
   │   GENERATES     │──── S65
   │  GRADING SIGNAL │
   └─────────────────┘
          │
          ▼
   ┌─────────────────┐
   │    PRESENTS     │──── S66
   │   INFORMATION   │
   └─────────────────┘
```

FIG. 6

FIG. 7

MEAN SPEED ACROSS RANGE

|  | 10 | 20 | 30 | 40 | ... |
|---|---|---|---|---|---|
| 10 | - | X1 m/s² | X2 m/s² | X3 m/s² | ... |
| 20 | - | - | X4 m/s² | X5 m/s² | ... |
| 30 | - | - | - | X6 m/s² |  |
| 40 | - | - | - | - | ... |
| ... | ... | ... | ... | ... | - |

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080015754 A
- US 20090248240 A
- US 20090319129 A
- GB 2443645 A